# EUROPÄISCHE PATENTSCHRIFT

(11) **EP 1 590 291 B1**
(45) Veröffentlichungstag und Bekanntmachung des Hinweises auf die Patenterteilung: **04.10.2006**
(21) Anmeldenummer: 05700755.1
(22) Anmeldetag: 07.01.2005
(51) Int. Cl.: B66C 1/12

(54) **MITTEL ZUM ANSCHLAGEN ODER ZUM VERZURREN EINES GUTS UND INFORMATIONSMITTEL**
MEANS FOR FIXING OR LASHING A PRODUCT, AND INFORMATION MEANS
MOYEN POUR ACCROCHER OU FIXER UN OBJET, ET MOYEN D'INFORMATION

(30) Priorität: 08.01.2004 DE 102004001265
(43) Veröffentlichungstag der Anmeldung: 02.11.2005
(73) Patentinhaber: Spanset Inter AG, 8618 Oetwil am See (CH)
(72) Erfinder: DOHSE, Lars, 52076 Aachen (DE); GLASEN, Werner, 52134 Herzogenrath (DE)
(74) Vertreter: Cohausz & Florack
(86) Internationale Anmeldenummer: PCT/EP2005/000104
(87) Internationale Veröffentlichungsnummer: WO 2005/066061

(56) Entgegenhaltungen:
- DE-U1- 8 102 337
- DE-U1- 8 708 432
- DE-U1- 9 414 241

## Beschreibung

Die Erfindung betrifft ein Mittel zum Anschlagen, Sichern oder Verzurren eines Guts oder zum Sichern einer Person, wie Spanngurt, Tragegurt, Zugband, Seil und Auffanggurte oder desgleichen, mit einem daran befestigten Informationsmittel. Derartige Produkte werden beispielsweise zum Heben, Fördern und Befestigen von Transportgütern sowie zur Absturzsicherung von Personen eingesetzt.

Darüber hinaus betrifft die Erfindung ein Informationsmittel zur Befestigung an einem Anschlag- oder Verzurrmittel der zuvor genannten Art, welches dazu dient, dem Anwender wichtige produktspezifische Informationen über das zu verwendende Anschlag- oder Verzurrmittel zu geben. Solche Informationen können der Produktname, die maximale Belastbarkeit bzw. Traglast, eine Kurzanleitung etc. sein.

Die bisher verwendeten Informationsmittel bestehen in der Regel entweder aus einem unmittelbaren Aufdruck der Informationen auf das entsprechende Anschlag- oder Verzurrmittel oder aber aus einem bedruckten Gewebeband, welches seinerseits mit dem entsprechenden Anschlag- oder Verzurrmittel bzw. PSA-Produkt ("PSA" = Personen-Sicherungs-Ausrüstung) verbunden ist. Diese Verbindung kann beispielsweise durch Aufnähen erfolgen.

Nachteilig am beschriebenen Stand der Technik ist allerdings, dass im Laufe der Zeit durch die hohen Belastungen, insbesondere aufgrund von Reibung oder Dehnung, die auf das Anschlag- oder Verzurrmittel sowie PSA-Produkte einwirken, das Informationsmittel beschädigt werden oder sogar abreißen kann. Ferner führt ein Flattern eines nur einseitig befestigten Informationsmittels im Fahrtwind zu Ermüdungsbrüchen am Befestigungspunkt. Auch kann es im Laufe der Zeit zu einem Abrieb der bedruckten Bereiche kommen, wodurch die Informationen unleserlich oder zumindest schwer erkennbar werden. Dies wirkt sich negativ auf Anwendungsfreundlichkeit und Produktsicherheit aus.

Zudem dürfen oft sehr hochwertige, teure in Rede stehende Produkte in der Regel nicht mehr benutzt werden, sobald die betreffenden Informationen nicht mehr einwandfrei feststellbar sind. Besonders kritisch wirkt sich dies dann aus, wenn das Informationsmittel in Folge einer äußeren Belastung von dem jeweiligen Anschlag- oder Verzurrmittel bzw. von dem PSA-Produkt im Einsatz abgetrennt wird. Mit dem Verlust des Informationsmittels geht in der Regel der sofortige Verlust der Zulassung einher.

Aus der deutschen Gebrauchsmusterschrift DE 81 02 337 U1 ist ein Hebeband bekannt, dass ein erstes in eine Bügel- bzw. Gurtbandschlaufennaht eingenähtes Etikett aufweist, das die zum Hebeband gehörige Information trägt. Als Sicherung gegen einen vorzeitigen Verlust der für die Zulassung des Hebebandes benötigten Informationen ist in die betreffende Naht zusätzlich ein zweites Etikett eingenäht, das nur geringfügig über den Stoß des im Bereich der Naht umgeschlagenen Bandes vorspringt. Dieses zweite, wesentlich kürzere Etikett ist mit den für die Zulassung unbedingt erforderlichen Angaben versehen, die zur Verfügung stehen müssen, solange das Band eingesetzt wird. Dadurch, dass das zweite Etikett nur sehr kurz über den Nahtbereich hervorsteht, soll es durch den Wulst gegen eine Beschädigung geschützt sein, der durch das im Nahtbereich umgeschlagene Band gebildet ist. Problematisch an dieser Art des Schutzes ist jedoch, dass die Schutzwirkung des Wulstes nur über eine sehr kurze Länge gegeben und zudem abhängig von der Dicke des Wulstes ist. Sofern die Schutzwirkung überhaupt eintritt, steht daher auf dem Etikett nur eine geringe Fläche zur Verfügung, die in der Praxis nicht ausreicht, um die vorschriftsmäßige Kennzeichnung eines Hebebandes in einer unter Praxisbedingungen lesbaren Form gewährleisten zu können. Hinzu kommt, dass es auch bei diesem Stand der Technik dazu kommen kann, dass erstes und zweites Etikett aufgrund der im praktischen Einsatz dauernd auftretenden Bewegungen im Nahtbereich ermüden und vorzeitig von dem Hebeband abgetrennt werden.

Neben dem voranstehend erläuterten Stand der Technik ist aus dem deutschen Gebrauchsmuster DE 94 14 241 U1 ein gewebtes Chemiefaserband bekannt, bei dem eine Beschriftung vorgesehen ist. Die Beschriftung wird dabei von einer beschriftungsfreundlichen, bevorzugt aus Kunststoff bestehenden Grundierungsschicht getragen, die dabei unmittelbar auf das Gewebegrundmaterial des Bandes aufgebracht ist. Sie stellt eine sichere Verbindung zwischen dem Gewebegrundmaterial des Bandes und der Beschichtung her. Durch eine auf die Beschriftung aufgebrachte äußere Schutzschicht ist die Beschriftung zudem gegen eine Beschädigung durch von außen wirkende Kräfte geschützt. Eine entsprechende, auf der von der Beschriftung abgewandten Rückseite des Gewebegrundmaterials aufgebrachte zweite Schutzschicht schützt die Beschichtung vor Beschädigungen durch Belastungen, die von der Rückseite her auf das Band wirken.

Ausgehend vom voranstehend erläuterten Stand der Technik bestand die Aufgabe der Erfindung darin, ein Mittel der Eingangs genannten Art und ein entsprechend geeignetes Informationsmittel zu schaffen, mit dem auch unter harten Einsatzbedingungen gewährleistet ist, dass die vorschriftsmäßige Kennzeichnung über eine lange Zeit erhalten bleibt.

In Bezug auf das Mittel zum Anschlagen, Sichern oder zum Verzurren eines Guts, wie Spanngurt, Tragegurt, Zugband, Seil, Auffanggurt oder desgleichen, mit einem daran befestigten Informationsmittel ist diese Aufgabe erfindungsgemäß dadurch gelöst worden, dass das Informationsmittel aus mindestens einem Identifikationsmittel, einer Einlage von gegenüber dem Identifikationsmittel erhöhter Reißfestigkeit und einer Schutzumhüllung gebildet ist, die mindestens das Identifikationsmittel umgibt.

In Bezug auf das Informationsmittel zur Kennzeichnung eines Mittels zum Anschlagen, Sichern oder Verzurren eines Guts oder zum Sichern einer Person, wie Spanngurt, Tragegurt, Zugband, Seil, Auffanggurt oder desgleichen, besteht die erfindungsgemäße Lösung der oben genannten Aufgabe darin, dass es aus mindestens je einem Identifikationsmittel, einer Einlage mit gegenüber dem Identifikationsmittel erhöhter Reißfestigkeit und einer Schutzumhüllung gebildet ist, die mindestens das Identifikationsmittel umgibt, und unabhängig von dem Mittel als Einheit miteinander verbunden ist.

Das erfindungsgemäße Informationsmittel ist mehrlagig aufgebaut. Jeder der Lage des Informationsmittels ist dabei eine eigene Funktion zugeordnet, so dass die Lagen jeweils aus einem für ihre Funktion optimal geeigneten Material hergestellt sein können. So kann es sich bei dem als Informationsträger vorgesehenen Identifikationsmittel um ein Etikett handeln, das aus einem Material hergestellt ist, welches sich optimal bedrucken lässt und auch nach langem Gebrauch noch eine gute Lesbarkeit gewährleistet. Ebenso kann das Identifikationsmittel so ausgewählt werden, dass es optimal als Träger für eine optisch, elektronisch oder in sonstiger Form maschinenlesbare Information geeignet ist.

Die Auswahl des jeweiligen Identifikationsmittels kann dabei ohne Rücksicht auf eine bestimmte, zur dauerhaften Befestigung erforderliche Festigkeit erfolgen, da es durch die reißfeste Einlage gegen eine vorzeitige Zerstörung geschützt ist. So lassen sich für das Identifikationsmittel Materialien verwenden, die nur eine geringe Reißfestigkeit aufweisen. Die eine höhere Reißfestigkeit als das Identifikationsmittel aufweisende Einlage stellt auch in diesem Fall sicher, dass das Identifikationsmittel über die gesamte Einsatzdauer des entsprechend gekennzeichneten Hebemittels, Sicherungsmittels, Verzurrmittels oder PSA-Produkts sicher befestigt bleibt.

Auf der reißfesten Einlage liegt das Identifikationsmittel bei einer von außen wirkenden Belastung auf und wird von ihr gestützt. Die reißfeste Einlage nimmt so die insgesamt auf das Informationsmittel wirkenden Zug- und Biegekräfte auf und verhindert, dass die verschiedenen Lagen des Informationsmittels durch diese Kräfte beschädigt werden.

Der Schutz des Identifikationsmittels gegen Abrieb oder vergleichbare Einwirkungen von außen stellt schließlich die Umhüllung sicher, die um Identifikationsmittel und die Einlage gelegt ist. Diese Ummantelung schützt das Identifikationsmittel und die Einlage insbesondere gegen Witterungseinflüsse und direkten mechanischen Kontakt mit Fremdteilen. Da auch sie durch die reißfeste Einlage gegen eine Zerstörung bei auf sie wirkenden Zugkräften oder Biegekräften geschützt ist, kann das für die Schutzumhüllung verwendete Material aus einem weichen, witterungsbeständigen und Stöße dämpfenden Material gefertigt sein, ohne selbst eine hohe Reißfestigkeit zu besitzen.

Durch die erfindungsgemäße Ausgestaltung eines Informationsmittels ist somit sichergestellt, dass die produktspezifischen Informationen auch nach langer Zeit und bei regelmäßiger Anwendung erhalten und zu jederzeit gut lesbar sind. Die Lebenserwartung eines erfindungsgemäßen Informationsmittels ist daher deutlich erhöht. Im Ergebnis stellt die Erfindung sicher, dass ein erfindungsgemäß ausgestattetes Mittel zum Anschlagen, Verzurren und Sichern bis zum Erreichen in vorschriftsmäßiger Weise gekennzeichnet bleibt und die Gefahr eines vorzeitigen Verlustes der Zulassung aufgrund eines Unbrauchbarwerdens seiner Kennzeichnung auf ein Minimum reduziert ist.

Die Schutzumhüllung kann als Schlauch, Folie oder als Schutzbeschichtung ausgebildet sein und beispielsweise aus einem Kunststoff, insbesondere aus Polyethylen (PE) oder aus Polyvinylchlorid (PVC), bestehen. Dabei ist die Ummantelung bevorzugt durchsichtig, um das Identifikationsmittel ohne großen Umstand lesen zu können. Sie sollte auch UV-beständig sein, damit auch noch bei längerem Gebrauch ihre einwandfreie Funktion gewährleistet bleibt.

Um eine ausreichende Flexibilität zu gewährleisten, sollte in solchen Fällen, in denen die Ummantelung aus PE- oder PVC-Material gefertigt ist, die Dicke der Ummantelung höchstens einen Millimeter betragen. Als Optimum für die Materialdicke der Ummantelung haben praktische Versuche eine Dicke von 0,3 mm +/- 0,1 mm ergeben.

Die reißfeste Einlage besteht gemäß einer bevorzugten Ausführungsform aus einer technischen Textilie, insbesondere einem Gewebe, und ist insbesondere bandförmig ausgebildet, um bei vereinfachter Befestigung auch ein längeres Identifikationsmittel problemlos stützen zu können. Die separate Einlage erhöht die Steifigkeit und damit die Stabilität des Informationsmittels, was insbesondere einem Flattern des Informationsmittels im Fahrtwind entgegenwirkt und die Gefahr eines Ermüdungsbruchs des Identifikationsmittels deutlich reduziert.

Bei Verwendung einer insbesondere bandförmigen, aber auch anders geformten reißfesten Einlage kann auf jeder Seite zumindest ein Identifikationsmittel vorgesehen sein. So kann beispielsweise auf der Vorderseite der Einlage ein Etikett mit der Produktbezeichnung und den seitens der betreffenden Normen geforderten Produktinformationen, wie der maximalen Traglast, und auf der Rückseite ein Etikett mit einer Kurzanleitung zur Verwendung des Produkts befestigt sein. Ebenso ist es denkbar, auf der einen Seite der Einlage ein maschinenlesbares Identifikationsmittel anzuordnen, während auf der anderen Seite der Einlage ein Etikett als Identifikationsmittel vorgesehen ist, auf dem die erforderlichen Informationen im Klartext angegeben sind.

Das Etikett kann mit der Einlage vernäht, verklebt und/oder vernietet sein. Ebenso ist es denkbar, beidseitig der Einlage Abschnitte des Identifikationsmittels dadurch zu positionieren, dass bei einem band- oder streifenförmig ausgebildeten Identifikationsmittel ein mit den erforderlichen Informationen versehener Streifenabschnitt des Identifikationsmittels um die reißfeste Einlage gelegt wird, so dass der eine Streifenabschnitt sich in Richtung der Einlage auf der Vorderseite und der andere Etikettstreifenabschnitt sich auf der anderen Seite der reißfesten Einlage erstreckt.

Insbesondere dann, wenn das Anschlag-, Verzurrmittel oder das PSA-Produkt zumindest abschnittsweise aus einem textilen Werkstoff besteht, beispielsweise gewebt ist, kann das Informationsmittel auf einfache Weise durch Annähen auf ihm befestigt werden. Ein Annähen ist aber auch möglich, wenn das Anschlag- oder Verzurrmittel aus einem anderen Material besteht. Selbstverständlich ist es auch denkbar, dass das Informationsmittel mit dem Anschlag- oder Verzurrmittel verklebt und/oder vernietet ist. Dabei kann auch nur ein Ende des Informationsmittels mit dem Anschlag- oder Verzurrmittel verbunden sein. Letztlich kommt es nur darauf an, dass diese Verbindung auch hohen Belastungen standhält.

Wird ein maschinenlesbares Identifikationsmittel eingesetzt, so kann beispielsweise eine vereinfachte Materialverwaltung dadurch ermöglicht werden, dass der Standort des entsprechend gekennzeichneten Trag-, Sicherungs- oder Verzurrmittels mittels geeigneter Detektoren in einem Lager erfasst wird. Des Weiteren können mit Hilfe des Identifikationsmittels die Materialaus- und Materialrückgabe auf einfache Weise organisiert werden. Darüber hinaus können die gesetzlich vorgeschriebenen Angaben bei einer Kontrolle direkt in ein Datenverarbeitungssystem eingelesen und mit dort vorhandenen Solldaten abgeglichen werden.

Kostengünstig lässt sich ein maschinenlesbares Identifikationsmittel dadurch verwirklichen, dass es ein Transponder ist. Ebenso ist es jedoch auch denkbar, einen optisch abtastbaren Barcode oder andere maschinenlesbare Informationen auf das Identifikationsmittel aufzubringen.

Abhängig von der Art des jeweils eingesetzten Identifikationsmittels kann es zweckmäßig sein, dieses Identifikationsmittel direkt auf der Einlage zu befestigen. Auf diese Weise stellt die Einlage mit ihrer erhöhten Reißfestigkeit auch bei Identifikationsmitteln, die empfindlich gegen Zug- oder Druckbelastungen sind, sicher, dass seine dauerhafte Funktion gewährleistet ist. Zu diesem Zweck kann das Identifikationsmittel mit der Einlage fest verbunden, insbesondere vernäht, vernietet und/oder verklebt werden.

Insbesondere dann, wenn das Identifikationsmittel von der reißfesteren Einlage getragen wird oder als Etikett ausgebildet ist, kann es ausreichend sein, wenn nur ein Abschnitt des Informationsmittels mit dem Anschlag- oder Verzurrmittel direkt verbunden ist. Dies ist beispielsweise dann gegeben, wenn als Identifikationsmittel ein längliches Etikett eingesetzt wird, dass nur an einer Schmalseite mit dem jeweiligen Hebe-, Verzurr- oder Sicherungsmittel verbunden wird.

Das Identifikationsmittel kann mit einer Schutzschicht überzogen sein, um einer Beschädigung durch äußere Einflüsse vorzubeugen. Dabei kann die Schutzschicht durch einen flexiblen Kunststoff, zum Beispiel ein Silikon oder ein Polyurethan, gebildet sein, die den Bewegungen des Informationsmittels folgen kann.

Nachfolgend wird die Erfindung anhand einer ein Ausführungsbeispiel darstellenden Zeichnung näher erläutert. Es zeigen schematisch:
- Fig. 1: einen Endabschnitt eines Spanngurts in seitlicher Ansicht,
- Fig. 2: ein Informationsmittel in seitlicher Ansicht.

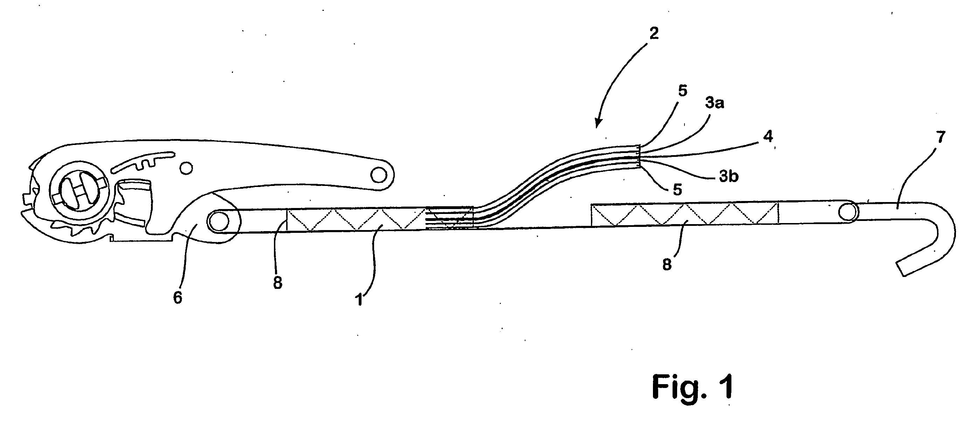

Der in Fig. 1 dargestellte, als Anschlag- oder Verzurrmittel dienende Spanngurt 1 bildet im Bereich seiner Enden zur Befestigung einer Ratsche 6 bzw. eines Hakens 7 eine Schlaufe, in dem sein eines Ende unter Ausbildung der Schlaufe umgelegt und auf den Endabschnitt des Spanngurts 1 gelegt worden ist. Damit sich diese Schlaufe auch bei hohen Belastungen nicht ungewollt löst, ist der Spanngurt in diesem Bereich vernäht. Die entsprechende Naht 8 ist in Fig. 1 durch eine gestrichelte Linie angedeutet.

Mittels derselben Naht 8 ist an dem Spanngurt 1 auch ein Informationsmittel 2 befestigt. Das Informationsmittel 2 umfasst eine bandförmige, gewebte Einlage 4, auf deren ein als Etikett ausgebildetes Identifikationsmittel 3a befestigt ist. Das Identifikationsmittel 3a ist mit der Produktbezeichnung und mit Informationen über die seitens der betreffenden Normen geforderten Produktinformationen, wie die maximale Traglast, bedruckt.

Auf der Rückseite der Einlage 4 ist ein weiteres nach Art eines Etiketts ausgebildetes Identifikationsmittel 3b befestigt, welches mit einer Kurzanleitung zur Verwendung des Spanngurtes bedruckt ist. Die Einlage 4 mit den beiden darauf befestigten Identifikationsmitteln 3a und 3b ist von einer Schutzumhüllung 5 aus strapazierfähigem und UVbeständigem Kunststoff umgeben, die eine Stärke von 0,3 mm aufweist. Da die Schutzumhüllung 5 durchsichtig ist, sind die darunter befindlichen Identifikationsmittel 3a und 3b gut lesbar.

Die Identifikationsmittel 3a,3b können dabei durch Abschnitte eines Streifens gebildet sein, der in Längsrichtung der reißfesten Einlage 4 gerichtet um deren dem Anschlagmittel 1 zugeordnetes Ende gelegt und dort gemeinsam mit der Einlage 4 mit dem Anschlagmittel 1 vernäht ist. Der auf diese Weise lose auf der Oberseite der Einlage 4 liegende Abschnitt des Streifens bildet dann das Identifikationsmittel 3a, während der entlang der Rückseite der Einlage 4 sich erstreckende Streifenabschnitt das Identifikationsmittel 3b bildet.

Das Informationsmittel 2 ist über die Naht 8 mit dem Anschlag- oder Verzurrmittel 1 lediglich an einem Ende verbunden. Auf diese Weise kann das Informationsmittel 2 zu beiden Seiten an den Spanngurt geklappt werden, so dass auch beim Heben, Fördern, Sichern oder Befestigen unterschiedlich geformter Transportgüter das Informationsmittel 2 immer gut sichtbar zugänglich ist.

Das zuvor beispielhaft beschriebene Anschlag- oder Verzurrmittel 1 und das entsprechende Informationsmittel 2 gewährleisten durch den stabilen und strapazierfähigen mehrlagigen Aufbau des Informationsmittels 2 auch nach langer Zeit noch eine gute Lesbarkeit der produktspezifischen Informationen. Außerdem wird durch den mehrschichtigen, soliden Aufbau auch die Gefahr eines Ermüdungsbruches durch Flattern des Informationsmittels 2 im Fahrtwind deutlich herabgesetzt. Insbesondere das Herausreißen wird durch die Unterlegung des Gurtbandes vermieden. Bislang war das Identifikationsmittelmaterial die Schwachstelle, da es einerseits gut bedruckbar und gleichzeitig in der Lage sein musste, hohe mechanische Belastungen zu ertragen. Insbesondere der Bereich der Einnähzone, an der das Identifikationsmittel beim Stand der Technik an das jeweilige Gurtband (Transport-, Sicherungs- oder Hebemittel) vernäht worden ist, war aufgrund der mit dem Vernähen einhergehenden Perforierung für ein Abreißen anfällig. Bei der Erfindung besteht dieses Problem nicht mehr, da die reißfeste Einlage das Identifikationsmittel stützt, so dass die im praktischen Einsatz auftretenden mechanischen Belastungen sich nicht mehr so stark auf das Material des Identifikationsmittels auswirken wie beim Stand der Technik.

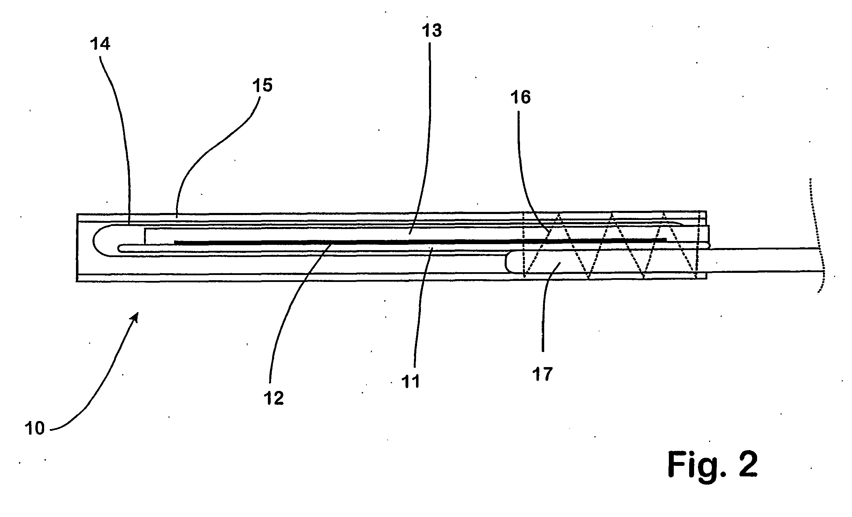

Das in Fig. 2 dargestellte Informationsmittel 10 weist eine reißfeste, bandförmige Lage 11 auf, die aus einem technischen Gewebe gefertigt ist, wie es beispielsweise zur Herstellung von Gurten etc. eingesetzt wird. Die reißfeste Einlage 11 trägt auf ihrer Oberseite ein Identifikationsmittel 12, das beispielsweise auf die Oberfläche der Einlage 11 aufgeklebt ist. Bei dem Identifikationsmittel 12 handelt es sich beispielsweise um einen handelsüblichen Transponder, der in geeigneter Weise so codiert ist, dass er eine eindeutige Identifikation des mit dem Informationsmittel 10 ausgestatteten Gurts, Tragezeuge etc. erlaubt. Mit Hilfe des Identifikationsmittels 12 lassen sich auf einfache Weise Lagerbestände, Einsatzort und -dauer und ähnliche für die ordnungsgemäße Verwendung von erfindungsgemäß gekennzeichneten Gütern relevanten erfassen, die von entscheidender Bedeutung für die sichere Verwendung dieser Güter sein können.

Zum Schutz gegen Beschädigungen durch von außen wirkende Belastungen ist das Identifikationsmittel 12 mit einer flexiblen Kunststoffschicht 13 überzogen, die beispielsweise durch ein auf die reißfeste Lage 11 aufgetragenes Polyurethan gebildet ist.

Um die reißfeste Einlage 11 mit dem Identifikationsmittel 12 und der Kunststoffschicht 13 ist in Längsrichtung der Einlage 11 ein wiederum etikettartiges Identifikationsmittel 14 gelegt, das die Ober- und Unterseite der Einlage abdeckt. Auf der jeweils außen liegenden Oberfläche des Identifikationsmittels 14 sind die notwendigen Produktinformationen ausgedruckt. Das Material des Identifikationsmittels 14 ist dabei so gewählt, dass es sich gut bedrucken und einfach um die Einlage 11 herumlegen lässt.

Über das Identifikationsmittel 14 ist als Schutzumhüllung 15 ein durchsichtiger, aus PVC gefertigter Schlauch gezogen, der das Identifikationsmittel 14 und mit ihm die reißfeste Einlage 11 mit dem Identifikationsmittel 12 und der Kunststoffschicht 13 von allen Seiten her abdeckt. Die reißfeste Einlage 11 mit dem Identifikationsmittel 12 und der Kunststoffschicht 13, und das die Einlage 11 in Längsrichtung gesehen umgebende Identifikationsmittel 14 sowie die Schutzumhüllung 15 bilden so einen Stapel von Materiallagen, die gemeinsam das Informationsmittel 10 bilden.

An seinem in Längsrichtung gesehenen einen Ende ist dieses Informationsmittel 10 mittels einer Naht 16 mit dem Ende eines Traggurtes 17 vernäht. Im praktischen Einsatz schützt die Schutzumhüllung 15 das Identifikationsmittel 14 und den Transponder 12 gegen äußere Einflüsse. Gleichzeitig stellt die reißfeste Lage sicher, dass das Informationsmittel 10 auch hohen mechanischen Belastungen standhalten und ein Abreißen vom Ende des Traggurtes 17 wirksam verhindert wird.

### BEZUGSZEICHEN

- 1: Anschlag- oder Verzurrmittel
- 2: Informationsmittel
- 3a: Identifikationsmittel
- 3b: Identifikationsmittel
- 4: Einlage
- 5: Schutzumhüllung
- 6: Ratsche
- 7: Haken
- 8: Naht
- 10: Informationsmittel
- 11: reißfeste, bandförmige Einlage
- 12: Identifikationsmittel
- 13: Kunststoffschicht
- 14: Identifikationsmittel
- 15: Schutzumhüllung
- 16: Naht
- 17: Traggurt

## Patentansprüche

1. Mittel (1) zum Anschlagen, Sichern oder Verzurren eines Guts oder zum Sichern einer Person, wie Spanngurt, Tragegurt, Zugband, Seil, Auffanggurt oder desgleichen, mit einem daran befestigten Informationsmittel (2), **dadurch gekennzeichnet, dass** das Informationsmittel (2) aus mindestens einem Identifikationsmittel (3a, 3b), einer Einlage (4) von gegenüber dem Identifikationsmittel (3a, 3b) erhöhter Reißfestigkeit und einer Schutzumhüllung (5) gebildet ist, die mindestens das Identifikationsmittel (3a, 3b) umgibt.

2. Mittel nach Anspruch 1, **dadurch gekennzeichnet, dass** es fest mit dem Informationsmittel (2) verbunden ist.

3. Mittel nach Anspruch 2, **dadurch gekennzeichnet, dass** es mit dem Informationsmittel (2) vernäht ist.

4. Mittel nach einem der Ansprüche 1 oder 2, **dadurch gekennzeichnet, dass** es mit dem Informationsmittel (2) verklebt und/oder vernietet ist.

5. Mittel nach einem der Ansprüche 1 oder 2, **dadurch gekennzeichnet, dass** das Identifikationsmittel (3a, 3b) lesbar ist.

6. Mittel nach Anspruch 5, **dadurch gekennzeichnet, dass** das Identifikationsmittel (2) maschinenlesbar ist.

7. Mittel nach einem der voranstehenden Ansprüche, **dadurch gekennzeichnet, dass** die Einlage (4) aus einer technischen Textilie, insbesondere einem Gewebe, besteht.

8. Mittel nach einem der voranstehenden Ansprüche, **dadurch gekennzeichnet, dass** die Einlage (4) bandförmig ausgebildet ist.

9. Mittel nach einem der voranstehenden Ansprüche, **dadurch gekennzeichnet, dass** die Einlage (4) beidseitig als Träger von Identifikationsmitteln (3a, 3b) dient.

10. Mittel nach einem der voranstehenden Ansprüche, **dadurch gekennzeichnet, dass** das Identifikationsmittel (3a, 3b) mit der Einlage (4) fest verbunden, insbesondere vernäht und/oder vernietet und/oder verklebt ist.

11. Mittel nach einem der voranstehenden Ansprüche, **dadurch gekennzeichnet, dass** nur ein Abschnitt des Informationsmittels (2) mit dem Anschlag- oder Verzurrmittel direkt verbunden ist.

12. Mittel nach einem der voranstehenden Ansprüche, **dadurch gekennzeichnet, dass** die Schutzumhüllung (5) ein Schlauch oder eine Folie ist.

13. Mittel nach einem der voranstehenden Ansprüche, **dadurch gekennzeichnet, dass** die Schutzumhüllung (5) aus Kunststoff, insbesondere aus Polyethylen (PE) oder aus Polyvinylchlorid (PVC), besteht.

14. Mittel nach einem der voranstehenden Ansprüche, **dadurch gekennzeichnet, dass** die Schutzumhüllung (5) durchsichtig ist.

15. Mittel nach einem der voranstehenden Ansprüche, **dadurch gekennzeichnet, dass** das Identifikationsmittel (3a, 3b) ein mit Informationen versehenes Etikett ist.

16. Mittel nach einem der voranstehenden Ansprüche, **dadurch gekennzeichnet, dass** das Identifikationsmittel (3a,3b) ein Transponder ist.

17. Mittel nach einem der voranstehenden Ansprüche, **dadurch gekennzeichnet, dass** das Identifikationsmittel (3a, 3b) von der Einlage getragen wird.

18. Mittel nach einem der voranstehenden Ansprüche, **dadurch gekennzeichnet, dass** die Schutzumhüllung als Schutzschicht ausgebildet ist, mit der das Identifikationsmittel (3a, 3b) überzogen ist.

19. Mittel nach Anspruch 18, **dadurch gekennzeichnet, dass** die Schutzschicht ein flexibler Kunststoff ist.

20. Mittel nach Anspruch 19, **dadurch gekennzeichnet, dass** der flexible Kunststoff ein Silikon oder Polyurethan ist.

21. Mittel nach einem der voranstehenden Ansprüche, **dadurch gekennzeichnet, dass** alle Lagen des Informationsmittels durch die Schutzumhüllung (5) umgeben sind.

22. Mittel nach einem der voranstehenden Ansprüche, **dadurch gekennzeichnet, dass** die Schutzumhüllung aus einem UV-beständigen Material besteht.

23. Informationsmittel (2) zur Kennzeichnung eines Mittels (1) zum Anschlagen, Sichern oder Verzurren eines Guts, wie Spanngurt, Tragegurt, Zugband, Seil, Auffanggurt oder desgleichen, **dadurch gekennzeichnet, dass** es aus mindestens je einem Identifikationsmittel (3a, 3b), einer Einlage mit gegenüber dem Identifikationsmittel (3a, 3b) erhöhter Reißfestigkeit und einer Schutzumhüllung (5), die mindestens das Identifikationsmittel (3a, 3b) umgibt, besteht und unabhängig von dem Mittel (1) zum Anschlagen, Sichern oder Verzurren eines Guts als Einheit miteinander verbunden ist.

## Claims

1. Means (1) for fastening, securing or clamping goods or for securing a person, such as a load strap, support strap, tie member, rope, safety harness or the like, with an information medium (2) attached thereto, **characterised in that** the information medium (2) consists of at least one identification medium (3a, 3b), an insert (4) having high tear strength relative to the identification medium (3a, 3b) and a protective casing (5), which surrounds at least the identification medium (3a, 3b).

2. Means according to Claim 1, **characterised in that** it is rigidly connected to the information medium (2).

3. Means according to Claim 2, **characterised in that** it is sewn to the information medium (2).

4. Means according to one of Claims 1 or 2, **characterised in that** it is glued and/or riveted to the information medium (2).

5. Means according to one of Claim 1 or 2, **characterised in that** the identification medium (3a, 3b) is readable.

6. Means according to Claim 5, **characterised in that** the identification medium (2) is machine-readable.

7. Means according to any one of the preceding claims, **characterised in that** the insert (4) consists of a technical textile, especially a fabric.

8. Means according to any one of the preceding claims, **characterised in that** the insert (4) is strip-shaped.

9. Means according to any one of the preceding claims, **characterised in that** the insert (4) acts on both sides as a carrier of identification media (3a, 3b).

10. Means according to any one of the preceding claims, **characterised in that** the identification medium (3a, 3b) is rigidly connected, especially sewn and/or riveted and/or glued, to the insert (4).

11. Means according to any one of the preceding claims, **characterised in that** only a portion of the information medium (2) is connected directly to the fastening or clamping means.

12. Means according to any one of the preceding claims, **characterised in that** the protective casing (5) is a tube or a film.

13. Means according to any one of the preceding claims, **characterised in that** the protective casing (5) is made of plastics material, especially of polyethylene (PE) or of polyvinyl chloride (PVC).

14. Means according to any one of the preceding claims, **characterised in that** the protective casing (5) is transparent.

15. Means according to any one of the preceding claims, **characterised in that** the identification medium (3a, 3b) is a label provided with information.

16. Means according to any one of the preceding claims, **characterised in that** the identification medium (3a, 3b) is a transponder.

17. Means according to any one of the preceding claims, **characterised in that** the identification medium (3a, 3b) is carried by the insert.

18. Means according to any one of the preceding claims, **characterised in that** the protective casing is formed as a protective layer with which the identification medium (3a, 3b) is covered.

19. Means according to Claim 18, **characterised in that** the protective layer is a flexible plastics material.

20. Means according to Claim 19, **characterised in that** the flexible plastics material is a silicone or polyurethane.

21. Means according to any one of the preceding claims, **characterised in that** all layers of the information medium are surrounded by the protective casing (5).

22. Means according to any one of the preceding claims, **characterised in that** the protective casing is made of a UV-resistant material.

23. Information medium (2) for marking a means (1) for fastening, securing or clamping goods, such as a load strap, support strap, tie member, rope, safety harness or the like, **characterised in that** it consists of at least one respective identification medium (3a, 3b), an insert having high tear strength relative to the identification medium (3a, 3b) and a protective casing (5), which surrounds at least the identification medium (3a, 3b), and is joined as a unit independently of the means (1) for fastening, securing or clamping goods.

## Revendications

1. Moyen (1) pour fixer, assurer ou accrocher un objet ou pour sécuriser une personne, comme une sangle de tension, une sangle de soutien, un lien en acier, un câble et une sangle de réception ou similaire, avec un moyen d'information (2) y fixé, **caractérisé en ce que** le moyen d'information (2) est formé d'au moins un moyen d'identification (3a, 3b), une pièce rapportée (4) de résistance à la déchirure augmentée par rapport au moyen d'identification (3a, 3b) et une enveloppe de protection (5), qui entoure au moins le moyen d'identification (3a, 3b).

2. Moyen selon la revendication 1, **caractérisé en ce qu'**il est relié fermement au moyen d'information (2).

3. Moyen selon la revendication 2, **caractérisé en ce qu'**il est cousu au moyen d'information (2).

4. Moyen selon la revendication 1 ou 2, **caractérisé en ce qu'**il collé et/ou riveté au moyen d'information (2).

5. Moyen selon la revendication 1 ou 2, **caractérisé en ce que** le moyen d'identification (3a, 3b) est lisible.

6. Moyen selon la revendication 5, **caractérisé en ce que** le moyen d'information (2) est lisible par machine.

7. Moyen selon l'une des revendications précédentes, **caractérisé en ce que** la pièce rapportée (4) consiste en un textile technique, en particulier un tissu.

8. Moyen selon l'une des revendications précédentes, **caractérisé en ce que** la pièce rapportée (4) est en forme de bande.

9. Moyen selon l'une des revendications précédentes, **caractérisé en ce que** la pièce rapportée (4) sert sur ses deux faces, comme support au moyens d'identification (3a, 3b).

10. Moyen selon l'une des revendications précédentes, **caractérisé en ce que** le moyen d'identification (3a, 3b) est solidement relié à la pièce rapportée (4), en particulier cousu et/ou riveté et/ou collé.

11. Moyen selon l'une des revendications précédentes, **caractérisé en ce que** seul un segment du moyen d'information (2) est directement relié au moyen de fixation ou d'accrochage.

12. Moyen selon l'une des revendications précédentes, **caractérisé en ce que** l'enveloppe de protection (5) est un passant ou une feuille.

13. Moyen selon l'une des revendications précédentes, **caractérisé en ce que** l'enveloppe de protection (5) consiste en une matière plastique, en particulier en polyéthylène (PE) ou en poly(chlorure de vinyle) (PVC).

14. Moyen selon l'une des revendications précédentes, **caractérisé en ce que** l'enveloppe de protection (5) est transparente.

15. Moyen selon l'une des revendications précédentes, **caractérisé en ce que** le moyen d'identification (3a, 3b) est une étiquette munie d'une information.

16. Moyen selon l'une des revendications précédentes, **caractérisé en ce que** le moyen d'identification (3a, 3b) est un transpondeur.

17. Moyen selon l'une des revendications précédentes, **caractérisé en ce que** le moyen d'identification (3a, 3b) est porté par la pièce rapportée.

18. Moyen selon l'une des revendications précédentes, **caractérisé en ce que** l'enveloppe de protection est formée comme une couche de protection, avec laquelle on revêt le moyen d'identification (3a, 3b).

19. Moyen selon l'une des revendications précédentes, **caractérisé en ce que** la feuille de protection est une matière plastique flexible.

20. Moyen selon l'une des revendications précédentes, **caractérisé en ce que** la matière plastique flexible est une silicone ou un polyuréthanne.

21. Moyen selon l'une des revendications précédentes, **caractérisé en ce que** toutes les couches du moyen d'information sont entourées par l'enveloppe de protection (5).

22. Moyen selon l'une des revendications précédentes, **caractérisé en ce que** l'enveloppe de protection consiste en un matériau résistant aux UV.

23. Moyen d'information (2) pour caractériser un moyen (1) pour fixer, assurer ou accrocher un objet, comme une sangle de tension, une sangle de soutien, un lien en acier, un câble et une sangle de réception ou similaire, **caractérisé en ce qu'**il consiste en au moins un moyen d'identification (3a, 3b), une pièce rapportée (4) de résistance à la déchirure augmentée par rapport au moyen d'identification (3a, 3b) et une enveloppe de protection (5), qui entoure au moins le moyen d'identification (3a, 3b), et sont reliés l'un avec l'autre, en tant qu'unité, indépendamment du moyen (1) pour fixer, assurer ou accrocher un objet.
